# FASCICULE DE BREVET EUROPEEN

(11) **EP 2 303 301 B1**
(45) Date de publication et mention de la délivrance du brevet: **12.09.2018**
(21) Numéro de dépôt: 09766062.5
(22) Date de dépôt: 27.05.2009
(51) Int. Cl.: A61K 36/54, A61K 36/53, A61K 36/45, A61P 13/00, A61K 31/11

(54) **COMPOSITIONS A BASE DE PLANTES ET UTILISATIONS**
ZUSAMMENSETZUNGEN AUF PFLANZENBASIS UND IHRE VERWENDUNGEN
PLANT-BASED COMPOSITIONS AND USES

(30) Priorité: 28.05.2008 FR 0853458
(43) Date de publication de la demande: 06.04.2011
(73) Titulaire: Addevista, 95650 Boissy-l'Aillerie (FR)
(72) Inventeur: PIERRON, Frédéric, F-86270 La Roche-Posay (FR); CORNU, Philippe, F-95650 Boissy l'Aillerie (FR)
(74) Mandataire: Becker, Philippe
(86) Numéro de dépôt international: PCT/FR2009/050991
(87) Numéro de publication internationale: WO 2009/153500

(56) Documents cités:
- WO-A-2004/014416
- WO-A-2007/109804
- DATABASE WPI Week 200878 Thomson Scientific, London, GB; AN 2008-N24921 XP002509878 "Chinese traditional external liniment for treating, e.g. headache or migraine, comprises camphor, methyl salicylate, turpenine oil, camphor wood oil, star anise oil, cinnamon oil, borneol, menthol, oleum eucalyptus and tea oil extract" & CN 101 181 425 A (WANG Y) 21 mai 2008 (2008-05-21)
- ARNAL-SCHNEBELEN B ET AL: "Essential oils in infectious gynaecological disease: a statistical study of 658 cases" INTERNATIONAL JOURNAL OF AROMATHERAPY, AROMATHERAPY PUBLICATIONS, HOVE, GB, vol. 14, no. 4, 1 janvier 2004 (2004-01-01), pages 192-197, XP004631342 ISSN: 0962-4562
- LOPEZ P ET AL: "Solid- and Vapor-Phase Antimicrobial Activities of Six Essential Oils: Susceptibility of Selected Foodborne Bcaterial and Fungal Strains" JOURNAL OF AGRICULTURAL AND FOOD CHEMISTRY, AMERICAN CHEMICAL SOCIETY. WASHINGTON, US, vol. 53, 1 janvier 2005 (2005-01-01), pages 6939-6946, XP002419484 ISSN: 0021-8561
- REID G: "THE ROLE OF CRANBERRY AND PROBIOTICS IN INTESTINAL AND UROGENITAL TRACT HEALTH" CRITICAL REVIEWS IN FOOD SCIENCE AND NUTRITION, BOCA RATON, FL, US, vol. 42, no. 3, SUPPL, 1 janvier 2002 (2002-01-01), pages 293-300, XP009081935 ISSN: 1040-8398
- HUSSAIN ET AL: "Chemical composition, antioxidant and antimicrobial activities of basil (Ocimum basilicum) essential oils depends on seasonal variations" FOOD CHEMISTRY, ELSEVIER SCIENCE PUBLISHERS LTD, GB, vol. 108, no. 3, 14 décembre 2007 (2007-12-14), pages 986-995, XP022461506 ISSN: 0308-8146

## Description

La présente invention concerne de nouvelles compositions à base de plantes et leurs utilisations, notamment comme complément ou additif alimentaire ou comme médicament. L'invention concerne plus particulièrement des compositions comprenant au moins un extrait de cannelle et un extrait de basilic, et leurs utilisations dans le domaine alimentaire, nutraceutique ou pharmaceutique, allopathique ou homéopathique. La présente invention concerne également l'utilisation d'un extrait de cannelle, seul ou en association avec au moins un autre extrait de plante, pour le traitement des infections urinaires.

### ARRIERE-PLAN TECHNOLOGIQUE DE L'INVENTION

Il existe de nombreux documents décrivant des préparations médicinales à base de plantes. Ainsi, par exemple, une base données, Database WPI Week 200878 de Thomson Scientific, mentionne une préparation traditionnelle chinoise à base d'huiles essentielles de plantes. Il s'agit d'une pommade destinée uniquement à un usage externe et préconisée pour le traitement de maux de tête, migraines, piqûres de moustiques ou enflures. Cette pommade comprend plus d'une soixantaine d'ingrédients, parmi lesquels, la quantité de l'huile de basilic et de l'huile de cannelle est insignifiante, la concentration de ces huiles n'excédant même pas 0,1 % du poids total de la pommade.

Il est connu dans la littérature que de nombreuses plantes possèdent des propriétés antibactériennes (voir notamment Cowan, Clinical Microbiology Reviews 12 (1999) 564). Ainsi, par exemple, Hussain et al., 2007 décrit des activités antimicrobiennes et anti-oxydantes de l'huile essentielle de basilic.

Une autre publication scientifique, Lopez et al., 2005, relate une étude de l'activité antimicrobienne de différentes huiles essentielles, qui est utilisée pour créer une atmosphère protectrice lors de conditionnement des aliments, permettant de minimiser la dégradation de leurs qualités organoleptiques. Ces huiles essentielles, parmi lesquelles figurent l'huile de cannelle et l'huile de basilic, n'ont jamais été testées dans leur intégralité. Seules leurs fractions solide ou gazeuse ont été testées séparément, à dilutions multiples. Aucune composition à base d'un mélange de cannelle et de basilic n'est décrite, ni suggérée dans ce document, ni son utilisation comme médicament ou complément alimentaire ou composé contre les infections urinaires.

La demande de brevet WO2004/014416 concerne une composition antimicrobienne comprenant entre autres des extraits de cannelle. Aucune utilisation de cette composition contre les infections urinaires n'est proposée dans ce document.

La demande de brevet WO2007/109804 concerne des extraits de différentes variétés de cannelle avec des fractions bien spécifiques obtenues par la technique de spectrométrie de masse. Une activité antibactérienne et antifongique de la cannelle est mentionnée dans ce document sans pour autant fournir de données standardisées ; de plus aucune composition à base de cannelle seule ou d'une combinaison de cannelle et de basilic n'est décrite ou suggérée dans ce document, et l'utilisation de la cannelle seule ou associée n'y est jamais décrite en tant que médicament ou complément alimentaire ou composé contre les infections urinaires.

Un autre document, Reid et al., 2002, étudie le rôle de la canneberge dans la prévention des infections intestinales et uro-génitales. Cependant, aucune composition à base de cannelle seule ou associée à la canneberge ou au basilic ou à toute autre plante n'est décrite dans ce document pour prévenir et encore moins traiter les symptômes d'une infection urinaire.

Les propriétés antibactériennes connues de certaines plantes sont liées à la présence de certains principes actifs contenus dans les plantes, tel que notamment les composés terpéniques, alcooliques, les proanthocyanidines, les composés phénoliques, les phénolméthyléthers, les aldéhydes aliphatiques et aromatiques. A l'exception de la canneberge, qui est largement utilisé en tant que complément alimentaire pour réduire la fréquence de survenue des épisodes de cystite et avec pour principaux composés les proanthocyanidines dont celles de type A, qui ont pour effet de réduire l'adhésion des bactéries E. coli sur la paroi vésicale et des voies urinaires dans leur ensemble, il n'existe pas d'autres compositions spécifiques à base d'une ou plusieurs plantes envisagées en tant que compléments alimentaires ou principes actifs de médicaments et ayant démontré une action bactéricide et/ou bactériostatique comparables à celle de certains antibiotiques sur les germes responsables des infections urinaires.

Sur un plan général, les plantes ont été utilisées pour le traitement des infections chez l'homme mais les études cliniques contrôlées sont rares et les quelques rapports établissant l'efficacité et la tolérance de compositions à base de plantes que l'on trouve sont généralement issus d'études non contrôlées et/ou non randomisées (Réf : Cowan, Clinical Microbiology Reviews 12 (1999) pages 575/577).
De même, des études bactériologiques comparant des huiles essentielles de référence et des antibiotiques de référence sur un groupe de germes représentatif des infections urinaires n'ont jamais été conduites.

### RESUME DE L'INVENTION

La présente invention concerne de nouvelles compositions à base de plantes telles que définies dans les revendications et leurs utilisations, notamment comme complément ou additif alimentaire ou comme médicament. L'invention concerne plus particulièrement des compositions comprenant au moins un extrait de cannelle et un extrait de basilic, et leurs utilisations dans le domaine alimentaire, nutraceutique ou pharmaceutique, allopathique ou homéopathique. Les compositions de l'invention sont utilisables notamment pour traiter, prévenir ou soulager les infections urinaires basses et hautes, ou pour améliorer la condition de patients atteints d'une infection urinaire.

### DESCRIPTION DETAILLEE DE L'INVENTION

La présente invention propose de nouvelles compositions à base de plantes telles que définies dans les revendications et leurs utilisations, notamment comme complément ou additif alimentaire ou comme médicament. Elle concerne également des compositions comprenant au moins un extrait de cannelle ou un extrait de cinnamaldéhyde et un extrait de basilic, et leurs utilisations dans le domaine alimentaire, nutraceutique ou pharmaceutique, allopathique ou homéopathique. Elle concerne en particulier une composition comprenant un extrait de cannelle sous forme d'huile essentielle pour le traitement ou la prévention d'une infection urinaire.
Un premier objet de l'invention concerne ainsi une composition comprenant, par rapport au poids total de la composition :
- de 1% à 80% en poids d'un extrait de cannelle ; et
- de 1% à 80% en poids d'un extrait de basilic, pour une utilisation pour traiter ou prévenir une infection urinaire ou soulager les symptômes de l'infection urinaire.

Un autre objet préféré de l'invention concerne une composition comprenant, par rapport au poids total de la composition :
- de 36 % à 81 % en poids d'un extrait de cannelle ; et
- de 19 % à 64 % en poids d'un extrait de basilic.

La composition comprend typiquement les deux extraits sous forme de mélange. L'extrait de cannelle comprend de préférence un extrait de Cinnamomum cassia (Tableau 1), l'extrait de basilic comprend de préférence un extrait de Ocimum basilicum L. et l'extrait de cinnamaldéhyde comprend de préférence un extrait de trans-cinnamaldéhyde. Par ailleurs, comme il sera discuté dans la suite du texte, la composition peut comprendre en outre d'autres ingrédients actifs et/ou des excipients acceptables sur le plan physiologique.

La divulgation concerne également l'utilisation d'un extrait de cannelle et d'un extrait de basilic traiter les infections urinaires. L'invention démontre en effet des propriétés antibactériennes remarquables d'extraits de cannelle, particulièrement adaptées pour le traitement de ce type d'infection. Ainsi, l'invention démontre pour la première fois, dans des tests standardisés, qu'un extrait de cannelle comprenant au moins 30% en poids de cinnamaldéhyde possède une action sur des germes caractéristiques des infections urinaires, plus puissante que certains antibiotiques. L'invention permet donc de proposer un traitement des infections urinaires utilisant un extrait de et de basilic soit sous forme de médicament, soit de complément alimentaire, à titre thérapeutique ou préventif, seul ou en complément d'autres traitements.

Un autre objet de la divulgation concerne un procédé de préparation d'une composition telle que définie ci-dessus, comprenant le mélange d'un extrait de cannelle, d'un extrait de basilic, et d'un extrait de canneberge. Un autre objet de l'invention réside dans l'utilisation d'une composition telle que définie dans la présente demande, pour la préparation d'un complément alimentaire ou médicament, en particulier pour le traitement ou la prévention d'une infection urinaire.
La présente invention concerne également une composition selon l'invention pour une utilisation pour traiter ou prévenir une infection urinaire ou soulager les symptômes de l'infection urinaire.

L'invention est utilisable notamment chez tout mammifère, en particulier animal ou humain, adulte ou enfant. Elle peut être utilisée pour le traitement ou l'amélioration de l'état physiologique de patients atteints d'infections à différents degrés de sévérité. Elle est particulièrement adaptée en cas de cystite.
Comme indiqué, l'invention résulte notamment de la mise en évidence surprenante que la cannelle associée au basilic, produit une composition ayant un profil d'activité antibactérien avantageux. La cannelle et le basilic sont des produits naturels, utilisés séparément dans l'alimentation. Cette utilisation ne permet pas de contrôler et de garantir la quantité d'actifs disponible à des fins curative ou préventive des infections urinaires. De plus, la cuisson par sa durée et la chaleur induite détruit de nombreuses doubles liaisons et les cycles aromatiques. Les propriétés liées à ces molécules sont inefficaces voire annihilées.
La cannelle a été retenue comme composé permanent de la composition selon l'invention pour être la plante significativement la plus bactéricide sur une série de germes jugés représentatifs pour être prépondérants et/ou virulents, et comparativement à un ensemble de plantes de référence et d'antibiotiques de référence. A titre d'exemple, l'effet antibactérien inattendu de la cannelle, démontré par les inventeurs (voir tableau 4) est même plus important que l'effet antibactérien obtenu avec certains antibiotiques (ex. le sulfaméthizole).

Les inventeurs ont également observé que l'activité du cinnamaldéhyde, qui est un ingrédient majoritaire de l'huile essentielle de Cinnamomum cassia, est particulièrement avantageuse dans les compositions selon l'invention, comme possédant l'une des activités bactéricides les plus spécifiques sur les mêmes germes étudiés.

Par conséquent, les compositions comprenant de la cannelle sous forme d'huile essentielle, et en particulier sous forme de l'huile essentielle de cinnamaldéhyde, de préférence trans-cinnamaldéhyde, sont des compositions préférées de l'invention (voir les tableaux 2 et 3). En termes de CMI, un extrait comprenant de la cannelle en association avec le basilic ou de la canneberge semble posséder une activité moins considérable qu'un extrait comprenant de la cannelle seule (tableau 2). Cependant, de manière surprenante, les inventeurs ont trouvé que les compositions comprenant de la cannelle en association avec le basilic ou de la canneberge, présentent des propriétés très avantageuses, qui permettent de préserver l'activité antibactérienne de la cannelle, d'élargir le spectre d'action et d'améliorer l'activité clinique du produit (anti-inflammatoire, antalgique, antiprurigineuse et antispasmodique).

Les compositions de l'invention sont utilisables notamment pour traiter ou prévenir les infections urinaires basses et hautes et/ou pour améliorer la condition de patients atteints d'une infection urinaire, et par extension toutes les infections localisées au niveau des organes ou des tissus où les germes étudiés peuvent être susceptibles de se développer et/ou être responsables de surinfection.

La cannelle est un arbre de la famille des Lauracées (famille des lauriers et avocatiers) atteignant 15 m de haut. Issue de Chine, on récolte son écorce. On obtient la cannelle en coupant des petites branches vieilles de 3 ans sur lesquelles on pratique des incisions longitudinales. En séchant, la fine couche intérieure de l'écorce dépouillée de son épiderme (couche externe) s'enroule sur elle-même, formant des bâtonnets friables de 7 à 8 cm de long et d'environ 1 cm de diamètre ayant la forme d'un tuyau (cannella en italien). A la fois épice et remède naturel, elle est considérée comme l'une des plantes médicinales les plus anciennes. L'écorce de Cannelle est une huile essentielle digestive (fermentation, gaz,), antispasmodique et antiseptique intestinal (diarrhée infectieuse, colite, oxyurose). Elle possède également une action tonique nerveuse (convalescence), cardiaque et respiratoire (infections à Gram (+) et (-)).

Il existe différentes variétés de Cannelle, parmi lesquelles on peut citer notamment : Cinnamomum acuminatifolium ; Cinnamomum acuminatissimum ; Cinnamomum acutatum ; Cinnamomum africanum ; Cinnamomum aggregatum ; Cinnamomum alainii ; Cinnamomum alatum ; Cinnamomum albiflorum ; Cinnamomum alcinii ; Cinnamomum alexei ; Cinnamomum alibertii ; Cinnamomum alternifolium ; Cinnamomum altissimum ; Cinnamomum ammannii ; Cinnamomum amoenum ; Cinnamomum amplexicaule ; Cinnamomum amplifolium ; Cinnamomum anacardium ; Cinnamomum andersonii ; Cinnamomum angustifolium ; Cinnamomum angustitepalum ; Cinnamomum antillarum ; Cinnamomum appelianum ; Cinnamomum arbusculum ; Cinnamomum archboldianum ; Cinnamomum areolatocostae ; Cinnamomum areolatum ; Cinnamomum areolatum ; Cinnamomum arfakense ; Cinnamomum argenteum ; Cinnamomumaromaticum (Cassia) ; Cinnamomum arsenei ; Cinnamomum asa-grayi ; Cinnamomum assamicum ; Cinnamomum aubletii ; Cinnamomum aureo-fulvum ; Cinnamomum australe ; Cinnamomum austro-sinense ; Cinnamomum austro-yunnanense ; Cinnamomum bahianum ; Cinnamomum bahiense ; Cinnamomum baileyanum ; Cinnamomum baillonii ; Cinnamomum balansae ; Cinnamomum bamoense ; Cinnamomum barbato-axillatum ; Cinnamomum barbeyanum ; Cinnamomum barlowii ; Cinnamomum bartheifolium ; Cinnamomum barthii ; Cinnamomum bazania ; Cinnamomum beccarii ; Cinnamomum bejolghota ; Cinnamomum bengalense ; Cinnamomum biafranum ; Cinnamomum bintulense ; Cinnamomum birmanicum ; Cinnamomum blumei ; Cinnamomum bodinieri ; Cinnamomum bonii ; Cinnamomum bonplandii ; Cinnamomum borneense ; Cinnamomum bourgeauvianum ; Cinnamomum boutonii ; Cinnamomum brachythyrsum ; Cinnamomum bractefoliaceum ; Cinnamomum burmannii ; Cinnamomum camphora (Camphor Laurel) ;Cinnamomum cassia (L.) Nees ; Cinnamomum caudiferum ; Cinnamomum chartophyllum ; Cinnamomum citriodorum ; Cinnamomum contractum ; Cinnamomum filipes ; Cinnamomum glanduliferum ; Cinnamomum glaucescens ; Cinnamomum ilicioides ; Cinnamomum impressinervium ; Cinnamomum iners ; Cinnamomum japonicum ; Cinnamomum javanicum ; Cinnamomum jensenianum ; Cinnamomum kotoense ; Cinnamomum kwangtungense ; Cinnamomum liangii ; Cinnamomum longepaniculatum ; Cinnamomum longipetiolatum ; Cinnamomum loureiroi (Saigon cinnamon) ; Cinnamomum mairei ; Cinnamomum micranthum ; Cinnamomum migao ; Cinnamomum mollifolium ; Cinnamomum oliveri ; Cinnamomum osmophloeum ; Cinnamomum parthenoxylon ; Cinnamomum pauciflorum ; Cinnamomum philippinense ; Cinnamomum pingbienense ; Cinnamomum pittosporoides ; Cinnamomum platyphyllum ; Cinnamomum porphyrium ; Cinnamomum reticulatum ; Cinnamomum rigidissimum ; Cinnamomum saxatile ; Cinnamomum septentrionale ; Cinnamomum subavenium ; Cinnamomum tamala (Malabathrum or Tejpat) ; Cinnamomum tenuipilum ; Cinnamomum tonkinense ; Cinnamomum triplinerve ; Cinnamomum tsangii ; Cinnamomum tsoi ; Cinnamomum validinerve ; Cinnamomum verum (Cinnamon or Ceylon cinnamon) ; et Cinnamomum wilsonii.

Parmi ces différentes variétés, on préfère tout particulièrement utiliser dans le cadre de la présente invention un extrait de Cinnamomum cassia (L.) Nees (Tableau 1), également désignée cannelle de Chine. Il est entendu que plusieurs variétés peuvent être utilisées, éventuellement en mélange.

La présente invention décrit pour la première fois les propriétés bactéricides et bactériostatiques de la cannelle qui permettent de réduire les doses ou la durée de traitement de certains antibiotiques ou encore de se substituer à eux, notamment en présence de germes dont Escherichia coli, Proteus mirabilis, Klebsiella pneumoniae, Pseudomonas aeruginosa, Pasteurella multocida et dont certains d'entre eux, ont développé des résistances vis-à-vis des antibiotiques.

La présente invention décrit également pour la première fois que les activités bactéricides et bactériostatiques de la cannelle sont préservées quand celle-ci est associée au basilic et/ou à tout autre extrait, en particulier les huiles essentielles de référence, malgré leur plus faible activité bactéricide et malgré une plus faible proportion de cannelle associée, et démontre également un véritable effet-dose sur son pouvoir bactéricide.

De manière surprenante, les inventeurs ont trouvé que l'utilisation d'huiles essentielles de plantes présente des activités beaucoup plus intéressantes par rapport à l'utilisation des extraits secs de plantes. Ainsi, l'extrait de plante sous forme d'huile essentielle, en particulier, l'huile essentielle de cannelle et/ou de basilic, est utilisé de manière préférentielle dans tous les modes de réalisation de l'invention décrits dans la présente demande. De manière encore plus préférentielle, on utilise une huile essentielle de cannelle contenant au moins 30 % de cinnamaldéhyde et/ou une huile essentielle de basilic.

De plus, la combinaison d'une huile essentielle de cannelle ou d'une autre huile contenant du cinnamaldéhyde et d'un extrait sec (quel qu'il soit à partir de cette invention, dans les cas où ces extraits secs témoignent de la présence de moisissures et/ou de colonies bactériennes) présente des caractéristiques très avantageuses. En effet, compte tenu de la présence de moisissures et de colonies de bactéries contenues, par exemple, dans l'extrait sec de canneberge, (celles-ci ont tendance naturellement à se développer dans le bouillon de culture), la présente invention démontre que la cannelle associée avec l'extrait sec de la canneberge permet d'une part de stériliser l'extrait de canneberge et de maintenir l'intégrité de ses composés, notamment les proanthocyanidines (PAC) et d'autre part de garder sa fonction bactéricide, et ainsi d'agir à la fois de manière curative et préventive sur les infections urinaires.

Le basilic est une plante de la famille des Lamiacées originaire du Vietnam. Le terme basilic est dérivé du grec « basilikon » signifiant «royal». Les anciens Grecs le réservaient aux souverains. Très odorant, le basilic est depuis toujours une herbe fort appréciée. Le basilic est abondamment utilisé dans les cuisines méditerranéennes, thaïlandaises, vietnamiennes et laotiennes. Cette plante trapue forme un petit buisson qui peut mesurer entre 20 et 60 cm de haut. Ses feuilles rondes ou lancéolées sont de couleur verte, plus ou moins foncée, rougeâtre ou pourpre. Fragiles, elles ont une saveur piquante qui atteint son maximum avant que des épis de fleurs blanches n'apparaissent aux extrémités des tiges. La saveur diffère selon les variétés ; elle peut rappeler le citron, le camphre, le jasmin, le clou de girofle, l'anis et le thym. La cueillette des feuilles doit s'effectuer avant la floraison, sinon elles perdent leur arôme. Le basilic est une huile essentielle digestive (spasmes digestifs, reflux gastro-oesophagien, aérophagie, diarrhées), pulmonaire (bronchite avec ou sans mucus, soulage l'asthme) et cutanée (psoriasis, ecchymoses, foulure, piqûre diverses).
Il existe différentes variétés de basilic, parmi lesquelles on peut citer notamment : Ocimum basilicum ssp ; Ocimum viride Willd. ; Ocimum americanum L. Synonymes ; O. stamineum Sims ; O. brachiatum Blume ; O. fluminense Vell. ; O. hispidiculum Schumach. & Thonn. ; O. incanescens Mart. ; O. pilosum Willd. ; O. citratum Rumph ; O. hispidum Lam. ; O. africanum Lour. ; O. graveolens A ; O. dichotomum Hochst. ex Benth. ; Ocimum basilicum L. Synonymes ; O. thyrsiflorum L. ; O. album L. ; O. médium Mill. ; O. bullatum Lam. ; O. integerrimum Willd. ; O. ciliatum Hornem. ; O. barrelieri Roth. ; O. Plectranthus barrelieri (Roth) Spreng. ; O. caryophyllatum Roxb., O. citrodorum Blanco. ; Ocimum canum ; Ocimum filamentosum Forssk. Synonymes : Becium filamentosum (Forssk.) Chiov. ; Ocimum adscendens Willd. ; Ocimum indicum Roth. ; Plectranthrus indicus (Roth.) Spreng. ; Ocimum cristatum Roxb. ; Ocimum exsul Collet & Helmsl. ; Ocimum gratissimum L. synonymes : O. petiolare Lam. ; O. urticifolium Roth ; O. viridiflorum Roth ; O. viride Willd. ; O. suave Willd. ; O. febrifugum Lindl. ; O. guineense Schumach. & Thonn. ; O. cillosum Weinm. ; O. paniculum Boj. ; O. anosurum Fenzl. ; Ocimum hispidus (Benth) Murata ; Ocimum kilimandscharicum Baker ex Gürke. ; Ocimum menthaefolium Benth. ; Ocimum tenuiflorum L. synonymes : O. sanctum L. ; O. monachorum L. ; O. inodorum Burm. ; O. tomentosum Lam. ; O. Lumnitzera tenuiflora (L.) Spreng. ; O. Plectranthus monoachorum (L.) Spreng. ; O. hirsutum Benth. ; O. villosum Roxb. ; O. album Blanco; O. Moschosma tenuiflora (L.) Heynh. ; O. nelsonii Zipp. ; O. virgatum Blanco et Ocimum ternifolius (D. Don) Kudo.

Parmi ces différentes variétés, on préfère tout particulièrement utiliser dans le cadre de la présente invention un extrait *d'Ocimum basilicum* L., plus préférentiellement Ocimum basilicum L. var basilicum. Il est entendu que plusieurs variétés peuvent être utilisées, éventuellement en mélange.

L'extrait de plante utilisé dans le cadre de la présente invention peut être tout type d'extrait, sec ou humide, préparé à partir de tout ou partie des plantes considérées selon des techniques généralement connues en elles-mêmes. Il peut s'agir de poudre totale, d'un extrait sec, d'une huile essentielle, ou de toute autre préparation issue de la plante.

La poudre totale de la plante (feuille, bois, fleur, fruit, racine, cône, tige, sommité fleurie, graine, racine, écorce, semence, tout ou partie de la plante) peut être préparée comme suit :
- on procède à un séchage naturel ou mécanique de la plante jusqu'à obtenir un taux d'humidité proche de 5 à 10 %. Les conditions opératoires diffèrent selon la nature de la partie à sécher (fleur, feuille, bois, cône, etc.).
- on effectue un broyage mécanique sans élévation de la température intrinsèque pour ne pas dénaturer les molécules thermosensibles, les plus fragiles.

Le broyage est typiquement effectué dans des conditions permettant l'obtention d'un broyat ayant une granulométrie comprise entre 150µm et 450µm environ, par exemple de l'ordre de 300 µm. Cette gamme de granulométrie correspond à la taille moyenne permettant à la fois une mécanisation du conditionnement et une surface de contact digestive importante, favorisant les échanges et donc l'assimilation à visée thérapeutique ou nutritive. Dans la mesure où l'oxydation de la poudre de plante est supérieure à celle de la plante entière, il est recommandé de n'entreprendre l'étape de broyage que le plus tardivement possible avant le conditionnement.

L'extrait sec résulte d'un processus d'extraction spécifique visant à ne conserver que les éléments dits actifs et supprimer mécaniquement ou chimiquement les éléments inertes ou sans rapport avec l'activité envisagée (cellulose, etc.). Ainsi, avec un extrait sec plus concentré qu'une poudre totale de plante, on réduit le volume ou la dose journalière indispensable au traitement, sans réduire la teneur en molécules actives. Les extraits secs peuvent être obtenus sans ou avec solvant(s) naturels ou chimiques. Dans un mode de mise en oeuvre particulier, les extraits secs peuvent être obtenus en traitant dans un premier temps la plante fraîche dans une solution vaporisable (éther, eau, alcool,...) par divers procédés d'extraction (macération, digestion, infusion, digestion, ou lixiviation). Dans un second temps, ces solutions sont évaporées jusqu'à obtenir une consistance fluide, molle ou sèche. En fonction de la concentration du solvant et du temps de contact on obtient un ratio r supérieur à 1 (plante fraîche/extrait sec). Dans le cas d'un produit sec, on peut le réduire en poudre.

Les huiles essentielles (ou essences végétales) correspondent au liquide concentré et hydrophobe des composés aromatiques essentiellement volatils d'une plante. C'est un mélange de molécules variées, comprenant en particulier des terpènes (hydrates de carbone non aromatiques) et des composés oxygénés dont les alcools, aldéhydes et/ou cétones.

Il existe plusieurs méthodes d'extraction des huiles essentielles, et notamment :
- l'entraînement à la vapeur ou l'hydro-distillation à partir de la plante fraîche ou sèche. La décantation permet de séparer la phase hydrophile de la phase hydrophobe ;
- l'extraction aux solvants volatils avec un extracteur du type Soxhlet et de l'hexane ou tout autre type de solvant. Ce procédé remplace l'enfleurage (méthode d'extraction par les graisses) ;
- l'extraction au CO2 supercritique, qui permet de ne pas utiliser et de ne pas retrouver de résidu de solvant ; ou encore
- l'extraction par expression à froid produisant une huile essentielle dénommée essence végétale. Cette technique est appliquée par mise en pressage à froid des bois, écorces, graines, noyaux, pépins, des algues, des champignons, des épices et toutes autres plantes (notamment les hespéridés : citron, orange, pomelos, mandarine).

Dans un mode de mise en oeuvre particulier de l'invention, on utilise un extrait de cannelle et/ou de basilic choisi parmi une poudre totale, un extrait sec ou une huile essentielle. De préférence, lorsque l'extrait d'une plante est une huile essentielle, l'extrait de ou des autres plantes associées peuvent (peut) être indifféremment une huile essentielle ou un extrait sec et selon un procédé de préparation dont un exemple est décrit dans la présente invention.

Dans un autre mode de mise en oeuvre particulier, le procédé de préparation d'une composition selon l'invention, permet d'associer une ou plusieurs huiles essentielles avec un extrait sec dans un produit final (gélule ou capsule) se présentant sous forme de poudre. De manière surprenante, cette association d'une ou plusieurs huiles essentielles et d'un extrait sec, confère au produit final sous forme de poudre, une meilleure homogénéité et permet ainsi un meilleur conditionnement du produit lors de sa fabrication industrielle.

La présente invention concerne également une gélule comprenant une huile essentielle de cannelle et une huile essentielle de basilic, microencapsulées à l'état de poudre, et associées à au moins un extrait sec de plante, de préférence un extrait sec de canneberge. Dans une variante, la présente invention concerne aussi une gélule comprenant une huile essentielle de cannelle, microencapsulée à l'état de poudre, et associée à au moins un extrait sec de plante, de préférence un extrait sec de canneberge.

L'extrait de cannelle peut être préparé à partir de la plante entière ou d'une partie de celle-ci choisie avantageusement parmi l'écorce, la feuille, la racine, le bois, la fleur et/ou les fruits. Un extrait préféré de cannelle est préparé à partir d'écorce. Il peut être préparé de 3 manières distinctes :
- Le totum de la plante : par broyage direct de l'écorce. La poudre est incorporée dans une unité galénique pour en maîtriser le dosage ;
- L'extrait sec, par concentration de l'écorce au ratio de 4/1 (ratio de 1/1 à un maximum de 25/1) ;
- L'huile essentielle par distillation à la vapeur d'eau par exemple afin de récupérer la phase hydrophobe qui est utilisée par voie externe ou interne à des doses adaptées en fonction de l'âge.

L'extrait de basilic peut être préparé à partir de la plante entière ou d'une partie de celle-ci choisie avantageusement parmi les feuilles, la partie aérienne de la plante fleurie ou non, la tige, les racines, semences et/ou graines. Un extrait préféré de basilic est préparé à partir des feuilles. Il peut être obtenu par les méthodes suivantes :
- Le totum de la plante : par broyage direct de la feuille sèche. La poudre est ensuite incorporée dans une unité galénique pour en maîtriser le dosage ;
- L'extrait sec, par concentration de la feuille au ratio de 4/1 (ratio de 1/1 à un maximum de 25/1) ;
- L'huile essentielle par distillation à la vapeur d'eau par exemple afin de récupérer la phase hydrophobe qui sera utilisée par voie externe ou interne à des doses adaptées en fonction de l'âge.

Dans un mode de réalisation particulier de la présente invention, l'extrait de basilic est un extrait de feuilles de basilic, de préférence sous forme d'extrait sec ou d'huile essentielle. Dans un mode particulièrement avantageux, l'extrait de basilic comprend des composés aldéhydes aliphatiques, cétones, alcools terpéniques, phénols, terpènes et autres.

Selon un autre mode de réalisation particulier de la présente invention, l'extrait de cannelle est un extrait d'écorce de cannelle, de préférence sous forme d'extrait sec ou d'huile essentielle. Dans un mode particulièrement avantageux, l'extrait de cannelle comprend les composés principaux comme le cinnamaldéhyde, le benzaldéhyde, le 4 éthyl gaiacol, l'isoeugénol et l'hydrocinnamaldéhyde.

Dans un mode de mise en oeuvre préféré, le rapport en poids entre l'extrait de cannelle et l'extrait de basilic dans les compositions de l'invention varie en fonction du type d'extrait.

Le rapport en poids entre l'extrait poudre de cannelle et de basilic est compris entre 0,1 et 10, de préférence entre 0,2 et 5, entre 0,2 et 3, typiquement entre 0,5 et 1, à l'exception des traitements à visée homéopathique pour lesquels aucun rapport n'est déterminé, mais correspondent à des doses variant entre 1/1000 et 1/10 de la dose usuelle recommandée pour les extraits poudre. Le rapport en poids entre l'extrait HE (huile essentielle) de cannelle et de basilic est compris entre 0,1 et 10, de préférence entre 0,2 et 5, entre 0,2 et 3, typiquement entre 0,5 et 3, à l'exception des traitements à visée homéopathique pour lesquels aucun rapport n'est déterminé, mais correspondent à des doses variant entre 1/1000 et 1/10 de la dose usuelle recommandée pour les extraits poudre. Le rapport en poids entre l'extrait HE de cannelle et l'extrait de canneberge est compris entre 0,01 et 10, de préférence entre 0,02 et 1, typiquement entre 0,05 et 0,3, à l'exception des traitements à visée homéopathique pour lesquels aucun rapport n'est déterminé, mais correspondent à des doses variant entre 1/1000 et 1/10 de la dose usuelle recommandée pour les extraits poudre. Des exemples particuliers ci-dessous montrent que le rapport 12/7 en poids entre un extrait de cannelle et un extrait d'une autre plante, de préférence basilic ou canneberge, est particulièrement efficace.

Dans un mode de mise en oeuvre préféré, l'extrait de cannelle représente de 1% à 80% du poids total de la composition, typiquement de 2 % à 80 %, plus préférentiellement de 30% à 55%, pour une teneur en excipients ou autres actifs disponible pour le complément. Dans un mode de mise en oeuvre particulier, l'extrait de cannelle représente de 1% à 20% pour les huiles essentielles, et de 30% à 55% pour les extraits secs.

Dans un mode de mise en oeuvre préféré, l'extrait de basilic représente de 1% à 80% du poids de la composition, typiquement de 2 % à 80 %, plus préférentiellement de 25% à 45%, pour une teneur en excipients ou autres actifs disponible pour le complément. Dans un mode de mise en oeuvre particulier, l'extrait de basilic représente de 1% à 20% pour les huiles essentielles, et de 30% à 55% pour les extraits secs.

Dans un mode de mise en oeuvre encore plus préféré, l'extrait de basilic est une huile essentielle et représente de 1 à 20 % du poids de la composition et/ou l'extrait de cannelle est une huile essentielle et représente de 1 à 20 % du poids de la composition.

Le pool de molécules sélectionnées et contenues dans le Cannelier de Chine (principalement cis-cinnamaldéhyde, trans-cinnamaldéhyde et hydroxycinnamaldéhyde, benzaldéhyde et isoeugénol) et dont les propriétés peuvent s'avérer essentielles dans la prise en charge de la cystite sont celles qui révèlent une action sur les germes bactériens et entérobactéries responsables des spasmes vésicaux et de l'inflammation des muqueuses du tractus urinaire.

L'Ocimum basilicum grâce à d'autres molécules, principalement le méthyl chavicol, les pinènes, le géraniol et géranial, l'eugénol, le thymol et le méthyl eugénol, possède le même spectre d'activité que le Cannelier de Chine sur Escherichia coli, Proteus mirabilis, Klebsiella pneumoniae et Pseudomonas aeruginosa, même si son action bactéricide et/ou bactériostatique est moins puissante sur ces germes, mais son association à la cannelle est avantageuse grâce à ses propriétés anti-infectieuse, antispasmodique et antalgique et son action sur divers autres germes souvent présents (Staphylococcus saprophyticcus, Entérobacteriaceae, Staphylococcus spp. et entérocoques) avec pour effet pour l'association de couvrir un spectre antibactérien plus large (cocci gram(+), bacilles Gram (+), bacilles Gram (-), entérobactéries, parasites, Candida spp. et Aspergillus [classification selon la norme Raisin du 16 11 2001].

Les doses physiologiques actives ont été déterminées afin de respecter les limites de sécurité optimale, soit une dose comprise typiquement pour chacun des ingrédients entre 1 mg et 50 mg sous forme d'huiles essentielles et entre 10 mg et 600 mg sous forme d'extraits secs.

Le dosage et le protocole peuvent être adaptés selon l'application recherchée et la composition. Typiquement, les compositions de l'invention sont utilisées à une fréquence et une quantité permettant la prise d'une dose quotidienne d'extrait de cannelle et de basilic comprise pour chacun des extraits, entre :
- 1 mg et 50 mg, plus préférentiellement entre 2 mg et 30 mg, pour les huiles essentielles ;
- 10 et 600 mg, plus préférentiellement entre 60 mg et 400 mg, pour les extraits secs.

A titre d'exemple spécifique, les doses suivantes peuvent être utilisées :
- Cinnamomum cassia à 12 mg par jour, sous forme d'huiles essentielles ou à 180 mg matin et soir, sous forme d'extrait sec ;
- Ocimum basilicum à 7 mg par jour, sous forme d'huiles essentielles ou à 280 mg matin et soir, sous forme d'extrait sec.

Il est entendu que les proportions respectives des ingrédients dans la composition finale peuvent varier et être adaptées par l'homme du métier en fonction du nombre d'ingrédients actifs présents, du type d'extrait, de la forme galénique, de l'utilisation recherchée.

La composition selon l'invention recense les activités spécifiques essentielles (antibactérienne, anti-inflammatoire, antiseptique, antispasmodique, bactériostatique, gramicide et antiseptique urinaire spécifique) et accessoires (analgésique-anesthésique, antioxydant-anti radicalaire, antipyrétique, anti Gram (+) anti Gram (-), candidicide et décongestionnant).

La composition selon l'invention peut être associée à un ou plusieurs autres ingrédients actifs ou excipients acceptables sur un plan physiologique. Parmi les ingrédients actifs supplémentaires, on peut citer tout principe actif, notamment un ou plusieurs extraits de plantes, choisis selon les propriétés désirées et la destination de la composition.

De manière avantageuse, on utilise un ou plusieurs ingrédients actifs supplémentaires présentant une action antispasmodique, antibactérienne générale, antibactérienne spécifique E. coli, entérobactéricides et Cocci Gram(+)icide bacille Gram(-)icide, anti-inflammatoire (notamment de l'arbre urinaire), antalgique, anti-infectieuse, antiprurigineuse, bactéricide, décongestionnante, diurétique, immunostimulante et/ou myorelaxante des fibres lisses.
A titre d'exemple, la composition de base peut comprendre en outre un ou plusieurs extraits choisis parmi Eugenia caryophyllata, Satureia montana, Thymus vulgaris, Perilla frutescens, Bursera delpechiana, Pelargonium graveolens, Citrus limon, Rosmarinus officinalis, ou encore pour les activités accessoires, Fagara schinifolia, Illicium verum, Myristica fragrans, Pimpinella asinatum, Artemisia dracunculus.
Une composition préférée au sens de l'invention comprend au moins :
- un extrait de cannelle,
- un extrait de basilic et
- un extrait de canneberge.
L'extrait de Cranberry (Canneberge, en particulier Vaccinium macrocarpon L.) est avantageusement préparé à partir des baies, notamment des baies matures. Il s'agit typiquement d'une poudre totale, qui peut être préparée par broyage direct de la baie. La poudre est alors typiquement incorporée dans une unité galénique pour en maîtriser le dosage. L'extrait utilisé peut également être un extrait sec, au ratio compris entre 1/1 et 50/1, typiquement de l'ordre de 10/1. Le solvant préféré pour l'extraction est l'éthanol, typiquement mis en oeuvre à un taux de 20 % à 40%.
L'intérêt de cette association se place surtout dès les derniers jours du traitement de la crise et sur la fréquence de survenue des épisodes.
Parmi les ingrédients supplémentaires qui peuvent être présents dans les compositions de l'invention en se rajoutant ou en se substituant à la cannelle, on peut citer des fruits, légumes, épices riches en proanthocyanidines comme l'avocat, la prune, la cacahuète, le curry, le cacao (réf. Gu, L., Kelm, M., Hammerstone, JF., Beecher, G., Holden, J., Haytowitz, D., Gebhardt, S., and Prior, R.L. - Concentrations of proanthocyanidins in common foods and estimations of normal consumption. J. Nutr., 2004, 134(3), 613-617).
On peut également citer des composés choisis parmi les vitamines, acides aminés, minéraux, et oligo-éléments. Des composés oligoéléments et minéraux utilisables sont notamment le cuivre (anti-infectieux), le sélénium (diurétique, anti-radicalaire), le magnésium (améliore les échanges cellulaires, diurétique), le zinc (intervenant dans les processus cicatriciels tissulaires), le phosphore (intervenant dans les réactions cellulaires), le molybdène (dont la carence entraine un déficit d'excrétion urinaire), le pro-carotène, le calcium, le bore. Parmi les vitamines, on peut citer notamment la vitamine A, indispensable à la structure cellulaire, la vitamine B1, impliquée dans la régulation l'équilibre cellulaire en sucres, la vitamine B6, utile au fonctionnement du système immunitaire, la vitamine C, antioxydant, la vitamine D, pour favoriser l'absorption du P et du Ca, ou encore la vitamine E, qui intervient dans le bon fonctionnement cellulaire. Il convient de prévoir ou non d'ajouter un ou plusieurs acides aminés favorisant par leur métabolisation une action complémentaire et/ou synergique.

Une composition préférée selon la divulgation comprend ainsi :
- un extrait de cannelle ;
- un extrait de basilic ;
- un excipient acceptable sur le plan physiopathologique ;
- de manière facultative, un extrait de canneberge ; et
- de manière facultative une vitamine, un minéral ou un oligoélément.

Une autre composition particulière selon la divulgation comprend :
- un extrait de cannelle ;
- un extrait de basilic ;
- un excipient acceptable sur le plan physiopathologique ; et
- une vitamine, un minéral ou un oligoélément.

Les compositions selon l'invention peuvent être des compositions pharmaceutique, cosmétique, nutraceutique ou alimentaire. Elles peuvent être administrées selon différentes voies et protocoles d'application. Ainsi, elles peuvent être adaptées à une administration orale, vaginale, rectale, topique, aérienne, systémique, par injection, etc. Dans ce contexte, différentes formes galéniques sont possibles, comme notamment des formes solides, semi-solides, souples, liquides, vaporisées ou pressurisées, vaporisations et diffusions ultrasoniques.

Parmi les formes galéniques solides, les compositions de l'invention peuvent se présenter sous forme de bâton, cachet, comprimé, enrobé ou non, effervescent, soluble, orodispersible, pelliculé, gastrorésistant ou à libération modifiée, capsule à enveloppe molle, dragée, gélule (capsules à enveloppe dure : HPMC, algues, cartilage, os) pelliculée ou non, gastrorésistante ou non, gomme ou pâte à mâcher, granule, granulé, pilule, pastille, poudre orale ou pour application topique, poudre lyophilisée soluble dans un liquide chaud ou froid, tablette, suppositoire ou ovule.

Parmi les formes galéniques souples, les compositions de l'invention peuvent se présenter sous forme de dispositif transdermique, de vernis souples.

Parmi les formes galéniques semi-solides, les compositions de l'invention peuvent se présenter sous forme de cataplasme, crème, emplâtres médicamenteux, gel, émulsion, pâte, huile ou pommade.

Parmi les formes galéniques liquides, les compositions de l'invention peuvent se présenter sous forme d'émulsion buvable (peut être en goutte), de suspension buvable (peut être en goutte), d'huiles de macération, de teinture officinale, de teinture mère, d'huile végétale en topique, liniment, liquide oral, lotion, mousse, sirop.

Parmi les formes galéniques pressurisées ou pneumatiques, les compositions de l'invention peuvent se présenter sous forme de nébuliseur, inhalateur (par exemple pressurisé à valve doseuse ou à poudre) et diffuseur sonique.

Selon la forme galénique utilisée, différents excipients ou véhicules acceptables sur le plan physiologique, essentiellement inertes, peuvent être présents dans les compositions de l'invention, comme des solutions isotoniques, tamponnées, salines, des sels, gommes, sucres, agents stabilisants, épaississants, édulcorants, gélifiants, tensio-actifs, etc.
Dans le cas de gélules ou capsules, on utilise typiquement du stéarate de magnésium et/ou de la silice colloïdale et/ou de la maltodextrine ou de la silice organique ou diverses huiles de macération ou d'expression obtenues par pressage à froid ou à chaud. Par ailleurs, l'enveloppe de ces capsules ou gélules peut être constituée de gélatine d'origine animale, végétale ou de poisson, d'algues, d'hydroxypropyl méthylcellulose (HPMC) et de cellulose. Elles peuvent être revêtues d'une couche gastrorésistante ou non et être modifiées pour effectuer une libération prolongée dans le tractus digestif.
Les compositions selon l'invention peuvent également se présenter sous forme de complément ou additif alimentaire, du type alicament (poudre à diluer, barre, ampoule, jus, etc.).
Ainsi, l'invention concerne une composition telle que définie ci-dessus à titre de médicament.
L'invention concerne également une composition telle que définie ci-dessus à titre d'agent cosmétique.
L'invention concerne également une composition telle que définie ci-dessus à titre de complément ou additif alimentaire.
L'invention concerne également une composition telle que définie ci-dessus à titre de spécialité homéopathique.

La présente divulgation concerne également un procédé de préparation d'une composition selon l'invention, telle que décrite ci-dessus, qui comprendrait une étape de mélange entre un extrait de cannelle et un extrait de basilic et, le cas échéant, un ou plusieurs autres extraits de plantes, en particulier de canneberge.

Un autre objet de la présente invention concerne l'utilisation de la composition de l'invention pour la préparation d'un médicament ou d'un complément alimentaire pour le traitement ou la prévention d'une infection urinaire.
Un autre objet particulier de la présente invention concerne l'utilisation d'un extrait de cannelle sous forme d'huile essentielle et d'un extrait de basilic pour la fabrication d'une composition destinée à traiter ou prévenir une infection urinaire.

La présente invention concerne également une composition comprenant un extrait de cannelle et de basilic sous forme d'huile essentielle pour le traitement d'une infection urinaire.

Un autre objet de la présente divulgation concerne une méthode pour traiter une infection urinaire, comprenant l'administration à un patient d'une quantité efficace d'un extrait de cannelle sous forme d'huile essentielle, en association avec un extrait de basilic.

Compte tenu des propriétés biologiques de la cannelle ou de la combinaison des extraits de cannelle avec le basilic et/ou la canneberge, les compositions de l'invention présentent une action antibactérienne adaptée à la prise en charge des infections chez les mammifères, notamment les humains, et peuvent être utilisées à titre curatif et/ou préventif, seules ou en combinaison ou alternance avec d'autres traitements. Les compositions de l'invention sont particulièrement adaptées au traitement des infections par entérobactéries affectant le tractus urinaire comme Escherichia coli, Klebsiella pneumoniae, Proteus mirabilis, Serratia et Pasteurella multocida et plus rarement associées à d'autres bactéries ou levures comme Chlamydia trachomatis, Mycoplasma hominis et Candida spp.
L'invention est particulièrement adaptée au traitement des infections de l'appareil urinaire, des voies basses ou hautes, et notamment de la cystite, en particulier de la cystite aigüe simple ou de la cystite aiguë compliquée. L'invention est également utilisable par exemple dans le cas de mycoses seules ou associées.
Comme indiqué ci-dessus, l'invention est utilisable pour la prise en charge de patients atteints de telles infections, ou pour améliorer l'état physiologique de personnes sujettes à de telles infections (ou qui ne souhaitent pas être traitées par des antibiotiques en première intention et/ou pour lesquelles les antibiotiques ne leur sont pas indiquées), ou bien afin de réduire les doses, en raison des intolérances et de l'émergence de résistance à ces produits. L'invention peut être utilisée seule, en traitement de première intention, ou en combinaison ou alternance avec d'autres traitements, notamment avec les antibiotiques. Elle permet de réduire la sévérité de l'infection, de bloquer son développement ou de la supprimer totalement. Elle permet de faciliter la défense de l'organisme contre l'infection. Elle est utilisable chez l'humain, mais également chez tout mammifère.
D'autres aspects et avantages de la présente invention apparaîtront à la lecture des exemples qui suivent, qui doivent être considérés comme illustratifs et non limitatifs.

### EXEMPLES

### Exemple 1 : Préparation de comprimés, capsules ou gélules

Les compositions suivantes sont préparées, adaptées à une administration orale. L'extrait de basilic utilisé est une poudre totale préparée par broyage. L'extrait de cannelle utilisé est une poudre totale préparée par broyage. L'extrait de canneberge utilisé est une poudre totale préparée par broyage.

### Comprimé ou gélule par voie orale 1 (2 plantes)

140 mg de poudre de Ocimum basilicum
90 mg de poudre de Cinnamomum cassia
Excipients pour comprimé ou pour gélule (qsp 100%)
Ce comprimé ou gélule est typiquement utilisé à raison de 1 à 3 prises quotidiennes de 1 à 2 comprimés ou gélules.

D'autres comprimés, capsules ou gélules similaires peuvent être produits dans lesquels les proportions respectives des ingrédients varient dans les gammes suivantes, le total ne pouvant dépasser 100% :
- poudre de Ocimum basilicum : 36 à 56%
- poudre de Cinnamomum cassia : 36 à 56%
- excipients : 4 à 28%

### Comprimé ou gélule par voie orale 2 (3 plantes)

140 mg de poudre de Ocimum basilicum
90 mg de poudre de Cinnamomum cassia
126 mg de poudre de Vaccinium macrocarpon
Excipients pour comprimé ou pour gélule (qsp 100%)
Ce comprimé ou gélule est typiquement utilisé à raison de 1 à 3 prises quotidiennes de 1 à 2 comprimés ou gélules.

D'autres comprimés, capsules ou gélules similaires peuvent être produits dans lesquels les proportions respectives des ingrédients varient dans les gammes suivantes, le total ne pouvant dépasser 100% :
- poudre de Ocimum basilicum : 30 à 60%
- poudre de Cinnamomum cassia : 15 à 50%
- poudre de Vaccinium macrocarpon : 30 à 50%
- excipients qsp 100%

### Exemple 2 : Préparation de gélules

Les compositions suivantes sont préparées, adaptées à une administration orale. L'extrait de basilic utilisé est une huile essentielle préparée par distillation à la vapeur d'eau. L'extrait de cannelle utilisé est une huile essentielle préparée par distillation à la vapeur d'eau également.
Gélules ou capsules de cannelle (illustratif) 5 à 30 mg d'huile essentielle de Cinnamomum cassia Excipients pour capsule ou pour gélule (qsp 100%)
Cette gélule ou capsule est typiquement utilisée à raison de 1 à 3 prises par jour.

### Gélules ou capsules de deux plantes

12 mg d'huile essentielle de Cinnamomum cassia
7 mg d'huile essentielle de Ocimum basilicum
Excipients pour capsule ou pour gélule (qsp 100%)
Cette gélule ou capsule est typiquement utilisée à raison de 1 à 3 prises par jour.
D'autres gélules similaires peuvent être produites dans lesquelles les proportions respectives des ingrédients varient dans les gammes suivantes, en pourcentage du poids total en ingrédients actifs :
- huile essentielle de Cinnamomum cassia : 25 à 90%
- huile essentielle de Ocimum basilicum : 30 à 75%
Cette gélule ou capsule est typiquement utilisée à raison de 1 à 3 prises quotidiennes.

### Granules ou microgranules homéopathiques à deux plantes

14 µg à 1,4 mg huile essentielle de Cinnamomum cassia
9 µg à 0,9 mg huile essentielle de Ocimum basilicum
Excipients pour granule ou microgranule (qsp 100%)
Posologie granule : 3 à 7 granules par jour pendant 10 jours, renouveler 3 fois. Posologie microgranule : 1 tube par jour pendant 10 jours, renouveler 3 fois.

### Exemple 3 : Procédés de préparation (encapsulation) d'une composition comprenant une étape de mélange entre deux ou trois ingrédients actifs, dont un extrait de cannelle et/ou un extrait de basilic sous forme d'huiles essentielles avec un extrait sec de canneberge

Les excipients sont choisis pour leur fonction en tant qu'agents de charge, émulsifiants et stabilisateurs. Une partie de la maltodextrine de la formule et l'ingrédient actif n°1 (par exemple, la canneberge) sont mis en oeuvre dans un équipement permettant une fluidisation de ces deux poudres. Cette étape permet d'effectuer un mélange homogène de celles-ci. La mise en oeuvre de l'ensemble des autres ingrédients, ingrédient actif n°2 et/ou ingrédient actif n°3 et le reste des excipients, est assurée dans un autre process permettant d'obtenir une solution homogène avant stabilisation. Cette solution est alors associée progressivement au mélange de poudres de maltodextrine et de l'ingrédient n°1 (la canneberge) en fluidisation. Cet ajout progressif de la solution permet d'obtenir des granules homogènes contenant en proportion identique une part de poudre initialement en fluidisation et une part de la solution déshydratée.

### Exemple 4 : Etude de l'activité antimicrobienne d'huiles essentielles de cinnamaldéhyde (HE) ou de cannelle (HE) associée ou non au basilic (HE) ou à un extrait sec de canneberge (ES) ou à une autre plante de référence versus antibiotiques de référence et autres plantes contre diverses souches bactériennes (Escherichia coli, Klebsiella pneumoniae, Proteus mirabilis, Pseudomonas aeruginosa et Pasteurella multocida)

### 1. Objectif et principe de l'étude :

Déterminer la CMI (Concentration Minimale Inhibitrice) d'huiles essentielles, versus antibiotiques et autres plantes.

### 2. Méthodologie et protocole standardisé :

La méthode est basée sur la détermination de la capacité de micro-organismes à produire une croissance visible dans un bouillon contenant des dilutions en série de l'agent antimicrobien. Des contrôles qualité sont réalisés sur au minimum deux souches bactériennes de référence parmi les 6 souches proposées dans la norme, et ces deux souches sont testées dans les mêmes conditions que les solutions bactériennes testées.

Les huiles essentielles sont vérifiées au niveau de leur quantité et de leur intégrité (étanchéité du contenant) et conservées à 4°C, à l'abri de la lumière. Chaque huile essentielle est testée à 7 concentrations et chaque concentration est testée 3 fois le même jour avec chacune des bactéries à 5. 10⁵ CFU/ml.

La détermination de la CMI des huiles essentielles est réalisée par microdilution en bouillon, conformément à la norme internationale NF EN ISO 20776-1 (Avril 2007) : Systèmes d'essais en laboratoire et de diagnostic in vitro - Sensibilité in vitro des agents infectieux et évaluation des performances des dispositifs pour antibiogrammes - Partie 1 : Méthode de référence pour la détermination de la sensibilité in vitro aux agents antimicrobiens des bactéries aérobies à croissance rapide impliquées dans les maladies infectieuses. L'ensemble des manipulations et préparations, est réalisé sous le poste de sécurité microbiologique, dans des conditions stériles. La préparation des différentes huiles en test est réalisée dans de l'eau physiologique additionnée de Tween 20 0.5%(v/v) afin de faciliter la solubilisation. La préparation des solutions antibiotiques est réalisée avec les mêmes solutions que celles utilisées pour les huiles.

Les plaques de microdilution préparées pour les essais, les témoins des essais, les témoins de croissance positifs et les témoins de croissance négatifs, doivent être inoculées dans les 30 minutes suivant la normalisation (10⁶ CFU/ml) de la suspension bactérienne afin de maintenir la concentration du nombre de cellules viables. 50µl de suspension bactérienne à 10⁶ CFU/ml sont ainsi déposés dans chacune des cupules contenant 50µl d'essai de chaque solution antibactérienne, préparation à base d'huile essentielle ou d'antibiotique ou bien contenant 50µl de bouillon (témoins de croissance positifs). Dénombrement des solutions bactériennes d'essai immédiatement après l'inoculation. Les plaques de microdilution et les géloses de dénombrement sont incubées à 37°C pendant 18 ± 2 heures.

### 3. Résultats :

### a. Contrôle de la viabilité du test

Les témoins de croissance, la pureté et le nombre de cellules dans l'inoculum sont vérifiés :
- témoins de croissance positifs : une turbidité est observée
- témoins de croissance négatifs : aucune turbidité n'est observée
- pureté : un seul type de colonies est observé sur les géloses de dénombrement
- nombre de cellules : le nombre de colonies sur les géloses est compris entre 20 et 80 (valeur minimale).
Le test n'est validé que si ces 4 points sont tous vérifiés, si le degré de croissance dans chaque cupule du contrôle qualité est comparable à celui des témoins de croissance positifs et si la CMI de l'antibiotique pour les bactéries témoins se trouve dans les plages de référence.

### b. Récapitulatif des résultats (voir Tableaux 2, 3, 4 et 5)

La qualité des résultats de tous les tests présentés dans ces tableaux sont vérifiés.

**Tableau 2 :** Etude de l'activité antimicrobienne d'huile essentielle de cinnamaldéhyde (HE) ou de cannelle (HE) associée ou non au basilic (HE) ou à un extrait sec de canneberge (ES) contre diverses souches bactériennes (Escherichia coli, Klebsiella pneumoniae, Proteus mirabilis).

Les compositions principales de la présente invention, à savoir le cinnamaldéhyde, la cannelle seule ou associée au basilic ou à la canneberge démontrent leur puissante activité bactéricide puisqu'elles inhibent toutes complètement la croissance des 3 types de souches représentés par Escherichia coli, Klebsiella pneumoniae et Proteus mirabilis dans des ordres de grandeurs compris entre 203 mg/L et 786 mg/L (voir tableau 2).

**Tableau 3 :** Etude comparative de l'activité antimicrobienne d'huile essentielle de cannelle (HE) versus d'autres huiles essentielles de référence contre diverses souches bactériennes (Escherichia coli, Klebsiella pneumoniae, Proteus mirabilis) présente les données suivantes :
Une détermination de la CMI de 8 huiles essentielles pour 3 souches bactériennes (Escherichia coli, Klebsiella pneumoniae et Proteus mirabilis) a permis de mettre en évidence la plus forte activité bactéricide de la cannelle sur l'ensemble des 3 germes étudiés devant un groupe compact de 5 plantes, Melaleuque, Origan, Sarriette, Clou de girofle et Ajowan. Aucune des plantes testées comparativement à la cannelle ne démontre un bon niveau d'activité général sur les 3 germes les plus représentatifs des infections urinaires. Or, dans les cas de survenue d'infections urinaires à l'hôpital ou lors d'infections urinaires récidivantes, Proteus mirabilis et Klebsiella pneumoniae deviennent plus fréquents avec respectivement 9,5% et 8% à l'hôpital, 13% et 11% pour les récidives.

**Tableau 4 :** Etude de l'activité antimicrobienne d'huile essentielle de cannelle (HE) associée ou non au basilic (HE) ou à des huiles essentielles de références versus des antibiotiques de référence contre diverses souches bactériennes (Escherichia coli, Klebsiella pneumoniae, Proteus mirabilis, Pseudomonas aeruginosa).

La cannelle, du fait de sa puissante activité bactéricide démontrée sur la triade d'entérobactéries que sont E. coli, K. pneumoniae et P. mirabilis démontre que cette activité bactéricide est bien supérieure à celle du sulfaméthizole, comparable à celle de l'amoxicilline sur K. pneumoniae et au moins comparable à celle des trois antibiotiques étudiés sur P.aeruginosa, amoxicilline, nitrofurantoïne et sulfaméthizole.

Les travaux publiés par Kern, Frimodt-Møller N, Espersen démontrent que le sulfaméthizole possède une CMI sur E.coli qui varie en fonction de la résistance des souches étudiées, une souche sensible correspondant à une CMI de 0,5 mg/L, une souche moyennement sensible à une CMI de 128 mg/L, une souche résistante à une CMI de 512 mg/L et une souche très résistante à une CMI > 2048 mg/L (Kern MB, Frimodt-Møller N, Espersen. F Urinary concentrations and urine ex-vivo effect of mecillinam and sulphamethizole. Clin Microbiol Infect 2004 ; 10 ; 54-61 et Kern MB, Frimodt-Møller N, Espersen F. Effects of Sulfamethizole and Amdinocillin against Escherichia coli Strains (with various Susceptibilities) in an Ascending Urinary Tract Infection Mouse Model. Antimicrobial Agents and Chemotherapy, Mar 2003, p.1002-1009).

Compte tenu des résultats obtenus par le sulfaméthizole pour E. coli dans l'étude bactériologique comparative à la cannelle (CMI > 512 mg/L), ceci démontre que la cannelle non seulement a démontré son activité bactéricide puissante contre E. coli, mais de surcroît sur des souches résistantes. La cannelle démontre également qu'elle peut maintenir une CMI optimale en remplaçant 30% de sa dose initiale par une autre huile, quel que soit le potentiel bactéricide de la plante associée.

**Tableau 5** : Etude de l'activité antimicrobienne d'huile essentielle de cannelle (HE) associée ou non au basilic (HE) suivant différents rapports de dose contre diverse souches bactériennes (Escherichia coli, Klebsiella pneumoniae, Proteus mirabilis).

**Tableau 6** : Etude de l'activité antimicrobienne d'huile essentielle de cannelle (HE) associée ou non au basilic (HE) suivant différents rapports de dose versus antibiotique de référence (ciprofloxacine) contre une souche bactérienne (Pasteurella multocida).

**Tableau 1 : Rapport d'analyse Huile essentielle Cinnamomum cassia, colonne polaire.**

| | TR [min] | Aire [%] |
|---|---|---|
| 1 alpha pinène | 3.02 | 0.12 |
| 2 camphène | 3.63 | 0.08 |
| 3 beta pinène | 4.35 | 0.04 |
| 4 cinéole 1,8 | 6.46 | 0.046 |
| paracymène | 8.54 | 0.04 |
| 7 alpha copaène | 16.19 | 0.22 |
| 8 benzaldéhyde | 17.05 | 0.87 |
| 9 beta caryophyllène | 19.82 | 0.10 |
| 10 alpha humulène | 22.47 | 0.21 |
| 12 alpha terpinéol | 23.81 | 0.09 |
| 14 safrole | 25.20 | 0.07 |
| 16 hydroxycinnaldéhyde | 25.85 | 0.06 |
| 19 cis cinnamaldéhyde | 29.78 | 0.32 |
| 22 oxyde de caryophyllène | 32.36 | 0.09 |
| 23 trans cinnamaldéhyde | 34.43 | 73.69 |
| 24 acétate de cinnamyle | 35.01 | 0.11 |
| 28 o methoxycinnamaldehyde | 37.28 | 0.09 |
| 29 eugénol | 38.11 | 2.85 |
| 30 alcool cinnamique | 41.87 | 0.20 |
| 31 coumarine | 45.32 | 1.70 |
| | | 100.00 |

**Tableau 2**

| **Souches** | **E. coli** | **K. pneumoniae** | **P. mirabilis** |
|---|---|---|---|
| **Produits tests (CMI en mg/L)** | | | |
| **Cannelle (HE)** | **203** | **405** | **405** |
| **Cinnamaldéhyde (HE)** | **205** | **410** | **410** |
| **Cannelle (HE)/Basilic (HE) 12/7** | **393** | **786** | **393** |
| **Cannelle (HE)/Canneberge (ES) 12/7** | **405** | **405** | **405** |

**Tableau 3**

| **Souches** | **E. coli** | **K. pneumoniae** | **P. mirabilis** |
|---|---|---|---|
| **Produits tests (CMI en mg/L)** | | | |
| **Cannelle (HE)** | **<203** | **405** | **<203** |
| **Ajowan (HE)** | **1410** | **1410** | |
| **Basilic (HE)** | **5988** | **11975** | **11975** |
| **Clou de girofle (HE)** | **1636** | | **1636** |
| **Ho-Wood, Shiu (HE)** | **2685** | **2685** | **1343** |
| **Melaleuque (HE)** | **698** | **1396** | **5582** |
| **Origan (HE)** | **732** | | |
| **Sarriette (HE)** | **714** | **1427** | |

**Tableau 4**

| **Souches** | **E. coli** | **K. pneumoniae** | **P. mirabilis** | **P. aeruginosa** |
|---|---|---|---|---|
| **Produits tests (CMI en mg/L)** | | | | |
| **Cannelle (HE)** | **<203** | **405** | **<203** | **810** |
| **Cannelle (HE)/Basilic (HE) 12/7** | **393** | **786** | **393** | **1572** |
| **Cannelle (HE)/Clou de girofle (HE) 12/7** | **406** | **406** | **406** | **1624** |
| **Cannelle (HE)/Melaleuque (HE) 12/7** | **384** | **384** | **384** | **1535** |
| **Sulfaméthizole** | **>512** | **>512** | **>512** | **>512** |
| **Nitrofurantoïne** | **8** | **32** | **128** | **>256** |
| **Amoxicilline** | **16** | **>256** | **8** | **>256** |

**Tableau 5**

| **Souches** | **E. coli** | **K. pneumoniae** | **P. mirabilis** |
|---|---|---|---|
| **Produits tests (CMI en mg/L)** | | | |
| **Cannelle (HE)** | **<203** | **405** | **<203** |
| **Cannelle (HE)/Basilic (HE) 12/7** | **393** | **786** | **393** |
| **Cannelle (HE)/Basilic (HE) 30/7** | **398** | **398** | **398** |
| **Cannelle (HE)/Basilic (HE) 7/12** | **734** | | |

**Tableau 6**

| **(CMI en mg/L)** | ***P. multocida*** | ***P. aeruginosa*** | ***S. aureus*** |
|---|---|---|---|
| **Cannelle (HE) 100%** | **97** | ND | ND |
| **Cannelle (HE)/Basilic (HE) 12**/7 | 1**88** | ND | ND |
| **Antibiotique Ciprofloxacine** | 0,0078 | 0,25 | 0,25 |

## Revendications

1. Composition comprenant, par rapport au poids total de la composition :
- de 1% à 80% en poids d'un extrait de cannelle ; et
- de 1% à 80% en poids d'un extrait de basilic
ledit extrait étant une huile essentielle, un extrait sec ou une poudre totale de la plante, ladite composition étant utilisée pour traiter ou prévenir une infection urinaire ou soulager les symptômes de l'infection urinaire.

2. Composition pour une utilisation selon la revendication 1, dans laquelle l'extrait de cannelle comprend un extrait de Cinnamomum cassia (cannelle de Chine) ou Cinnamonum verum (cannelle de Ceylan).

3. Composition pour une utilisation selon la revendication 1 ou 2, dans laquelle l'extrait de basilic comprend un extrait de Ocimum basilicum L.

4. Composition pour une utilisation selon l'une quelconque des revendications précédentes, **caractérisée en ce que** le rapport en poids de l'extrait de cannelle sur l'extrait de basilic est compris entre 0,1 et 10, de préférence entre 0,2 et 5.

5. Composition pour une utilisation selon l'une quelconque des revendications précédentes, **caractérisée en ce que** l'huile essentielle de basilic représente de 1 à 20 % du poids de la composition et/ou **en ce que** l'huile essentielle de cannelle représente de 1 à 20 % du poids de la composition.

6. Composition pour une utilisation selon l'une quelconque des revendications précédentes, **caractérisée en ce qu'**elle comprend en outre un ou plusieurs autres ingrédients actifs.

7. Composition pour une utilisation selon la revendication 6, **caractérisée en ce que** l'un au moins des autres ingrédients actifs est un extrait de plante, de préférence un extrait de canneberge.

8. Composition pour une utilisation selon l'une quelconque des revendications précédentes, **caractérisée en ce qu'**elle comprend en outre un composé choisi parmi une vitamine, un acide aminé, un minéral et un oligoélément.

9. Composition pour une utilisation selon l'une quelconque des revendications précédentes, **caractérisée en ce qu'**elle comprend en outre un excipient ou véhicule acceptable sur le plan physiologique.

10. Composition **caractérisée en ce qu'**elle comprend :
- un extrait de cannelle ;
- un extrait de basilic ; et
- un extrait de canneberge.

11. Composition ou composition pour une utilisation selon l'une quelconque des revendications précédentes, **caractérisée en ce que** la composition se présente sous forme de gélule, capsule, granule, comprimé, crème, gel, émulsion, huile, gomme, dispositif transdermique, ou pâte à mâcher.

12. Composition ou composition pour une utilisation selon l'une quelconque des revendications précédentes, **caractérisée en ce que** la composition est adaptée à une administration orale ou vaginale.

13. Composition selon la revendication 10, pour une utilisation en tant que médicament.

14. Utilisation de la composition selon l'une quelconque des revendications 1 à 12 pour la préparation d'un médicament ou d'un complément alimentaire pour le traitement ou la prévention d'une infection urinaire.

15. Gélule comprenant une huile essentielle de cannelle et une huile essentielle de basilic, microencapsulées à l'état de poudre, et associées à au moins un extrait sec de plante, de préférence un extrait sec de canneberge.

16. Utilisation selon la revendication 14, **caractérisée en ce que** ladite composition comprend au moins 30 % en poids de trans-cinnamaldéhyde.

17. Composition comprenant, par rapport au poids total de la composition :
- de 36% à 81% en poids d'un extrait de cannelle ; et
- de 19% à 64% en poids d'un extrait de basilic,
ledit extrait étant une huile essentielle, un extrait sec ou une poudre totale de la plante.

## Patentansprüche

1. Zusammensetzung, umfassend bezogen auf das Gesamtgewicht der Zusammensetzung:
- 1 Gew.-% bis 80 Gew.-% eines Zimtextrakts; und
- 1 Gew.-% bis 80 Gew.-% eines Basilikumextrakts,
wobei besagter Extrakt ein ätherisches Öl, ein Trockenextrakt oder ein Gesamtpulver der Pflanze ist, wobei besagte Zusammensetzung zur Behandlung oder Prävention einer Harnwegsinfektion oder Linderung der Symptome der Harnwegsinfektion verwendet wird.

2. Zusammensetzung für eine Verwendung gemäß Anspruch 1, wobei der Zimtextrakt einen Extrakt von Cinnamomum cassia (China-Zimt) oder Cinnamomum verum (Ceylon-Zimt) umfasst.

3. Zusammensetzung für eine Verwendung gemäß Anspruch 1 oder 2, wobei der Basilikumextrakt einen Extrakt von Ocimum basilicum L. umfasst.

4. Zusammensetzung für eine Verwendung gemäß einem der vorhergehenden Ansprüche, **dadurch gekennzeichnet, dass** das Gewichtsverhältnis von Zimtextrakt zu Basilikumextrakt zwischen 0,1 und 10, vorzugsweise zwischen 0,2 und 5 beträgt.

5. Zusammensetzung für eine Verwendung gemäß einem der vorhergehenden Ansprüche, **dadurch gekennzeichnet, dass** das ätherische Basilikumöl 1 bis 20 Gew.-% der Zusammensetzung darstellt und/oder dass das ätherische Zimtöl 1 bis 20 Gew.-% der Zusammensetzung darstellt.

6. Zusammensetzung für eine Verwendung gemäß einem der vorhergehenden Ansprüche, **dadurch gekennzeichnet, dass** sie außerdem einen oder mehrere andere aktive Bestandteile umfasst.

7. Zusammensetzung für eine Verwendung gemäß Anspruch 6, **dadurch gekennzeichnet, dass** wenigstens einer der anderen aktiven Bestandteile ein Pflanzenextrakt, vorzugsweise ein Moosbeerenextrakt ist.

8. Zusammensetzung für eine Verwendung gemäß einem der vorhergehenden Ansprüche, **dadurch gekennzeichnet, dass** sie außerdem eine Verbindung, ausgewählt aus einem Vitamin, einer Aminosäure, einem Mineral und einem Spurenelement, umfasst.

9. Zusammensetzung für eine Verwendung gemäß einem der vorhergehenden Ansprüche, **dadurch gekennzeichnet, dass** sie außerdem einen physiologisch verträglichen Arzneimittelträger oder ein physiologisch verträgliches Vehikel umfasst.

10. Zusammensetzung, **dadurch gekennzeichnet, dass** sie umfasst:
- einen Zimtextrakt;
- einen Basilikumextrakt; und
- einen Moosbeerenextrakt.

11. Zusammensetzung oder Zusammensetzung für eine Verwendung gemäß einem der vorhergehenden Ansprüche, **dadurch gekennzeichnet, dass** die Zusammensetzung als Gelatinekapsel, Kapsel, Granulat, Tablette, Creme, Gel, Emulsion, Öl, Gummi, transdermale Vorrichtung oder Kaumasse vorliegt.

12. Zusammensetzung oder Zusammensetzung für eine Verwendung gemäß einem der vorhergehenden Ansprüche, **dadurch gekennzeichnet, dass** die Zusammensetzung für eine orale oder vaginale Verabreichung geeignet ist.

13. Zusammensetzung gemäß Anspruch 10 für eine Verwendung als Medikament.

14. Verwendung der Zusammensetzung gemäß einem der Ansprüche 1 bis 12 zur Herstellung eines Medikaments oder einer Nahrungsergänzung für die Behandlung oder Prävention einer Harnwegsinfektion.

15. Gelatinekapsel, umfassend ein ätherisches Zimtöl und ein ätherisches Basilikumöl, die in Pulverform mikroverkapselt und mit wenigstens einem Pflanzentrockenextrakt, vorzugsweise einem Moosbeerentrockenextrakt assoziiert sind.

16. Verwendung gemäß Anspruch 14, **dadurch gekennzeichnet, dass** besagte Zusammensetzung wenigstens 30 Gew.-% trans-Zimtaldehyd umfasst.

17. Zusammensetzung, umfassend bezogen auf das Gesamtgewicht der Zusammensetzung:
- 36 Gew.-% bis 81 Gew.-% eines Zimtextrakts; und
- 19 Gew.-% bis 64 Gew.-% eines Basilikumextrakts,
wobei besagter Extrakt ein ätherisches Öl, ein Trockenextrakt oder ein Gesamtpulver der Pflanze ist.

## Claims

1. A composition comprising, compared to the total weight of the composition:
- from 1% to 80% by weight of an extract of cinnamon; and
- from 1% to 80% by weight of an extract of basil,
said extract being an essential oil, a dry extract or a total powder of the plant,
said composition being for use for treating or preventing a urinary infection or to relieve the symptoms of a urinary infection.

2. The composition for use according to claim 1, wherein the extract of cinnamon comprises an extract of *Cinnamomum cassia* (Chinese cinnamon) or Cinnamomum verum (Ceylan cinnamon).

3. The composition for use according to claim 1 or 2, wherein the extract of basil comprises an extract of *Ocimum basilicum* L.

4. The composition for use according to any one of the preceding claims, wherein the ratio by weight of the extract of cinnamon to the extract of basil is between 0.1 and 10, preferably between 0.2 and 5.

5. The composition for use according to any one of the preceding claims, wherein the essential oil of basil represents from 1% to 20% of the weight of the composition and/or wherein the essential oil of cinnamon represents from 1% to 20% of the weight of the composition.

6. The composition for use according to any one of the preceding claims, wherein it further comprises one or more other active ingredients.

7. The composition for use according to claim 6, wherein at least one of the other active ingredients is a plant extract, preferably an extract of cranberry.

8. The composition for use according to any one of the preceding claims, wherein it further comprises a compound selected from a vitamin, an amino acid, a mineral and an oligoelement.

9. The composition for use according to any one of the preceding claims, wherein it further comprises a physiologically acceptable excipient or carrier.

10. A composition comprising:
- an extract of cinnamon;
- an extract of basil; and
- an extract of cranberry.

11. The composition or composition for use according to any one of the preceding claims, wherein the composition is provided in the form of a gelatin capsule, capsule, granule, tablet, cream, gel, emulsion, oil, paste, a transdermal device, or chewable gum.

12. The composition or composition for use according to any one of the preceding claims, wherein the composition is adapted to oral or vaginal administration.

13. The composition according to claim 10, for use as a medicament.

14. Use of the composition according to any one of claims 1 to 12, to prepare a medicament or a dietary supplement to treat or prevent a urinary infection.

15. A gelatin capsule comprising an essential oil of cinnamon and an essential oil of basil, microencapsulated in powder state, and associated with at least one dry plant extract, preferably a dry extract of cranberry.

16. Use according to claim 14, wherein said composition comprises at least 30% by weight of trans-cinnamaldehyde.

17. A composition comprising, compared to the total weight of the composition:
- from 36% to 81% by weight of an extract of cinnamon; and
- from 19% to 64% by weight of an extract of basil,
said extract being an essential oil, a dry extract or a total powder of the plant.
